# EUROPEAN PATENT SPECIFICATION

(11) **EP 1 023 598 B1**
(45) Date of publication and mention of the grant of the patent: **13.07.2005**
(21) Application number: 98918336.3
(22) Date of filing: 16.04.1998
(51) Int. Cl.: G01N 31/22

(54) **CHEMICAL INDICATOR AND ITS USE**
CHEMISCHER INDIKATOR UND DESSEN VERWENDUNG
INDICATEUR CHIMIQUE ET SON UTILISATION

(30) Priority: 17.04.1997 US 44079 P
(43) Date of publication of application: 02.08.2000
(73) Proprietor: ETHICON, INC., Somerville, NJ 08876 (US)
(72) Inventor: SWANZY, James, A., Arlington, TX 76015 (US)
(74) Representative: Fisher, Adrian John
(86) International application number: PCT/US1998/007733
(87) International publication number: WO 1998/046994

(56) References cited:
- WO-A-96/33242
- WO-A-98/46279
- US-A- 4 444 879
- US-A- 4 938 860
- US-A- 5 139 957
- US-A- 5 571 568
- US-A- 5 708 141
- DATABASE WPI Section Ch, Week 198836 Derwent Publications Ltd., London, GB; Class J04, AN 1988-253593 XP002231185 & JP 63 184043 A (KURABO IND LTD), 29 July 1988 (1988-07-29)

## Description

### DETAILED DESCRIPTION OF INVENTION

### Technical Field

This invention relates to a composition for use as a chemical indicator, in particular, a composition useful in a hydrogen peroxide plasma sterilization treatment, and to a sheet for said treatment on which sheet said composition is deposited.

### Background Art

Since long ago, various kind of sterilization means have been applied to articles of every species such as disposable or recyclable medical apparatuses and food containers, and there have also been proposed indicators for conveniently distinguishing whether such a sterilization treatment has been made or not. For example, Japanese Patent Application Laid-Open (Kokai) No. 59-36172/1984 discloses an indicator for gas sterilization treatment with use of ethylene oxide; and Japanese Patent Application Laid-Open (Kokai) Nos. 61-287972/1986, Hei 5-43827/1993 and Hei 5-65441/1993 each disclose an indicator ink for electron beam sterilization. The latter group of Laid-Open (Kokai) Applications each mention an indicator wherein a pH indicator is used in combination with such a high molecular compound as is capable of generating hydrogen chloride when irradiated with electron beam.

On the other hand, as a means to conduct a sterilization treatment without adversely affecting medical equipments which are produced from various materials, there have recently been proposed and put into practice a hydrogen peroxide plasma sterilization method and an apparatus therefor (See: Japanese Patent Publication (Kokoku) Nos. Hei 2-62261/1990 and Hei 7-22693/1995). This sterilization method summarily comprises a step of contacting an article to be sterilized with hydrogen peroxide vapor under reduced pressure in an air-tight chamber, and then generating hydrogen peroxide plasma. This method can be said to be a very useful one, not only in that high sterilization efficiency is attained but also in that hydrogen peroxide is converted into water and oxygen which are quite harmless.

The above-mentioned Japanese Patent Publication (Kokoku) No. Hei 7-22693/1995 discloses a liquid-dispensing cassette to be used in a plasma sterilization apparatus which cassette is equipped with a cell for containing a hydrogen peroxide solution. It is further disclosed that said dispensing cassette may be equipped with an indicator strip having such color tone with which to detect the leakage of hydrogen peroxide solution from the liquid-containing cell. Said Publication does not concretely disclose, however, how to constitute the indicator strip.

### PROBLEMS WHICH THIS INVENTION INTENDS TO SOLVE

Also in putting a hydrogen peroxide plasma sterilization method into practice, it would be desirable that there is available such an indicator which can easily distinguish whether or not a sterilization treatment has been given to an article to be sterilized, as is employed in the aforementioned gas sterilization treatment with use of ethylene oxide or electron beam sterilization treatment. Thus, the object of this invention is to provide a chemical indicator composition (indicator ink, in particular) which can distinguish whether or not a hydrogen peroxide plasma sterilization treatment has been given to an article to be sterilized.

### MEANS TO SOLVE THE PROBLEMS

The aforementioned Japanese Patent Application Laid-Open (Kokai) No. Hei 5-65441/1993, for example, suggests that, when bisphenols and a substance such as triphenylsulfonium hexafluorophosphate which generates acid or free radical when irradiated with electron beam are compounded with an indicator ink which comprises a pH indicator and such a high molecular compound as generates hydrogen chloride when irradiated with electron beam, there can be improved color changeability of said ink when the ink is irradiated with electron beam. The inventors of the present invention, on the other hand, have found out that, when a dye belonging to a specific pH indicator is brought into contact with a system which comprises hydrogen peroxide and plasma derived from hydrogen peroxide and which does not generate hydrogen chloride, there occurs a certain color change, which can be both stabilized and rendered distinct when a certain organic amine is made to co-exist.

Thus, in order to achieve the above-mentioned object, this invention provides a composition for use as a chemical indicator which comprises both a dye which can change its color when contacted with at least one substance selected from the group consisting of hydrogen peroxide and plasma derived from hydrogen peroxide, and an organic amine compound which does not evaporate under ambient conditions.

This invention further provides a sheet used for distinguishing whether a hydrogen peroxide plasma sterilization treatment has been made or not, which sheet has, deposited on its substrate, both a layer containing such a composition (or indicator ink) and an overcoat layer which is provided on said layer.

This invention which has the aforementioned features makes it possible to clearly distinguish whether medical apparatuses, food containers and the like have undergone or not such a hydrogen peroxide plasma sterilization treatment, even after a certain time has passed from said treatment.

### EMBODIMENTS OF INVENTION

As for the dye, in the composition of this invention for use as a chemical indicator, which can change its color when contacted with at least one substance selected from the group consisting of hydrogen peroxide and plasma derived from hydrogen peroxide, there can be employed dyes of any kind so long as they make it possible, owing to their change in color before and after said contact, to clearly distinguish whether such a contact has been made or not. Typical examples of such dyes, although not restricted, are pH indicators which have a transition interval in a range from pH 5.5 -9.0. Concrete examples of such pH indicators include 1,2-dihydroxy anthraquinone (pH 5.5 ~6.8); dibromothymol sulfonphthalein (Bromothymol Blue: pH 6.0 ∼7.5); 5,8-quinolinequinon-8-hydroxy-5-quinolyl-5-imide (pH 6.0 ∼8.0); 3-amino-6-dimethylamino-2-methylphenazine hydrochloride (pH 6.8 ~8.0); phenolsulfonphthalein (Phenol Red: pH 6.8 ∼8.4); o-cresolsulfon phthalein (Cresol Red: pH 7.2 ∼8.8); m-cresolsulfon phthalein (pH 7.4 -9.0) and the like, and their derivatives. In practical use, two or more of these indicators may be combined with one another.

This invention is characterized in that the above dye is used along with an organic amine compound which does not evaporate under ambient conditions (concretely, at a room temperature at which the sterilization treatment is made). Any kind of such organic amine compounds are usable so long as they do not evaporate throughout sterilization treatment, in particular a low temperature sterilization treatment with use of hydrogen peroxide (See: for example, Japanese Patent Publication (Kokoku) No. Hei 2-62261/1990; Thus cited, this Publications constitute a part of the present invention), and so long as they can adjust pH of the composition to alkaline side. Examples of such organic amine compounds include mono higher aliphatic amine such as laurylamine, mono hydroxy higher aliphatic amine and triethanol amine. Among these, triethanol amine is preferably employed in consideration of compatibility with the dye used in this invention and compatibility with synthetic resin which may be included as vehible in the composition.

The composition of this invention can usually contain both a vehicle (including synthetic resin, solvent, and, if necessary, plasticizer as well), which is normally used for the preparation of printing ink, and additive (such as dispersant, stabilizer and thickening agent). As for resin, those derived from any kind of synthetic polymers are usable unless the resins are adversely affected in view of achievement of the object of this invention, when subjected to the aforementioned hydrogen peroxide plasma sterilization treatment, Concrete examples of such synthetic resins include phenolic resin, alkyd resin, urea resin, polyamide resin, acrylic resin and synthetic rubbers.

These synthetic resins may be suitably selected in accordance with substrate which is used in processing the composition of this invention into a sheet, and in consideration of how to apply the composition onto the substrate (or of printing method). In the case of indirect application (or offset printing) onto the substrate, for example, there can be employed acrylonitrile-butadiene-styrene resin which is capable of providing a composition having a considerably high viscosity (about 200 ∼2000 P). As such acrylonitrile-butadiene-styrene resin, there can be preferably employed, for example, ABS-800 Extender Base, Clear (Advance Process Supply, Chicago, U.S.A.) on the market. This ABS-800 is preferably employed together with glycerol and monoalkyl glycerol ethers such as 2-butoxyethanol either as a solvent as another vehicle or as an additive, and, under circumstances, also together with ultraviolet light absorber.

The above-mentioned composition may comprise 0.3 -10.0 % by weight of dye, 5 ∼15 % by weight of organic amine compound, 45 ∼70 % by weight of synthetic resin, 20 ∼33 % by weight of solvent (such as monoalkyl glycerol ethers) and 1 ~3 % by weight of glycerol, each on the basis of the total amount of the composition. As for substrate, polystyrene type one is preferably employed although not restrictively, when such an ABS resin as mentioned above is used.

From the above-explained composition of this invention, there can be conveniently produced a sheet which has, on its substrate, deposited at least said composition to form an indicator-function layer and an overcoat layer on said indicator-function layer. The overcoat layer may be formed from any components so long as the resulting layer is permeable to hydrogen peroxide vapor or plasma derived from hydrogen peroxide, and so long as said formed layer is transparent or semi-transparent such that the color change of dye can be seen through. Preferably, however, the overcoat layer is formed from a composition which contains those components to form the indicator-function layer, except dye and organic amine compounds but including ultraviolet light absorber instead, and, under circumstances, further including 0.3 ∼10 % by weight of wax (e.g., polyethylene wax) on the basis of the total amount of the composition.

As for ultraviolet light absorber, those of any species may be employed so long as they are normally used in this field and unless they have adverse effects in view of achievement of the object of this invention. For example, benzotriazole derivatives on the market are preferably used. Typical examples of said derivatives include Tinuvin™ type compounds having sunscreen property produced by Ciba-Geigy, which are to be employed singly or in combination of two or more of them.

The sheet comprising thus formed layers may take a strip-like form, or may be a part of a packaging bag for packing articles to be sterilized.

The indicator-function layer is normally formed on the surface of a breathing sheet which constitutes a part of packaging bag. When packaging bag is so transparent as to show its interior, indicator-function layer may be formed either on the inner side of breathing sheet, or on the inner side of laminate sheet, i.e., inside the packaging bag.

The above-mentioned composition of this invention is homogenized by such a known kneading method as is employed for the preparation of printing ink, and is then deposited on a substrate to form an indicator-function layer and, subsequently, an overcoat layer, in accordance with a known printing method, e.g., offset printing, flexographic printing or gravure printing, and, thus, the sheet of this invention can be produced

### EXAMPLES

In the following, this invention is explained in more detail with concrete examples. Percentage in the examples mean "% by weight" unless otherwise specified.

### Example 1 Formation of indicator-function layer and overcoat layer:

A composition having the following components

| | |
|---|---|
| ABS-800 Extender Base (Advance Process Supply) | 60 (%) |
| Triethanol amine | 10 |
| Glycerol | 2 |
| Phenol Red (including no acid) | 0.5 |
| 2-Butoxyethanol | 27.5 |
| Total | 100.0 |

was mixed and kneaded by a roll mill until the composition became homogeneous, and, thus, a composition for indicator was prepared.

Apart from the above, a composition having the following components

| | |
|---|---|
| ABS-800 Extender Base (Same as above) | 60 (%) |
| Dipsal (Sunscreen: made by Scher chemicals Inc.) | 2 |
| 2-Butoxyethanol | 38 |
| Total | 100 |

was mixed and kneaded by a roll mill until the composition became homogeneous, and, thus, a composition for overcoat was prepared.

Thus prepared composition for indicator and composition for overcoat were printed, in this order, on a white and opaque polystyrene sheet. Thus formed laminate sheet may be cut to a suitable size to be a chemical indicator strip.

### EFFECTS OF INVENTION

This invention provides a composition with which to clearly distinguish whether packed articles such as medical apparatuses have undergone a sterilization treatment or not.

## Claims

1. A sheet for distinguishing whether a hydrogen peroxide plasma sterilization treatment has been made, said sheet comprising a substrate, an indicator-function layer deposited on said substrate and a coating layer deposited on said indicator-function layer, wherein said indicator-function layer comprises a dye which can change its color when contacted with hydrogen peroxide or plasma derived from hydrogen peroxide, and an organic amine compound which does not evaporate under ambient conditions, selected from mono higher aliphatic amines, mono hydroxy higher aliphatic amines and triethanol amine, and wherein said coating layer is permeable to hydrogen peroxide vapor or plasma derived from hydrogen peroxide, and is further transparent or semi-transparent such that a change of color of the dye is visible through said coating layer.

2. A sheet according to claim 1 wherein the dye is a pH indicator which has a transition interval in the range from pH 5.5 to 9.0, and wherein the organic amine compound is triethanol amine.

3. A sheet according to claim 2 wherein the dye is phenol red.

4. A sheet according to any preceding claim wherein said coating layer is formed from a solvent selected from glycerol and mono alkyl glycerol ethers, and a synthetic resin which is compatible with said solvent.

5. A sheet according to claim 4 wherein said coating layer further comprises an ultraviolet light absorber and a wax.

6. A sheet according to claim 4 or claim 5, wherein the synthetic resin is acrylonitrile-butadiene-styrene resin.

## Patentansprüche

1. Schicht zum Erkennen, ob eine Wasserstoffperoxidplasmasterilisierungsbehandlung gemacht worden ist, wobei die Schicht ein Substrat, eine auf dem Substrat abgelegte Indikatorfunktionsschicht und eine auf der Indikatorfunktionsschicht abgelegte Deckschicht umfaßt, wobei die Indikatorfunktionsschicht einen Farbstoff, der bei Kontakt mit Wasserstoffperoxid oder aus Wasserstoffperoxid stammendem Plasma seine Farbe verändert, und eine organische Aminverbindung umfaßt, die unter Umgebungsbedingungen nicht verdampft, ausgewählt aus längerkettigen aliphatischen Mono-Aminen, längerkettigen aliphatischen Monohydroxy-Aminen und Triethanolamin, und wobei die Deckschicht für Wasserstoffperoxiddampf oder aus Wasserstoffperoxid stammendes Plasma durchlässig ist und weiterhin transparent oder semi-transparent ist, so daß eine Farbveränderung des Farbstoffs durch die Deckschicht sichtbar ist.

2. Schicht nach Anspruch 1, wobei der Farbstoff ein pH-Indikator ist, der ein Übergangsintervall im Bereich von pH 5,5 bis 9,0 hat, und wobei die organische Aminverbindung Triethanolamin ist.

3. Schicht nach Anspruch 2, wobei der Farbstoff Phenolrot ist.

4. Schicht nach einem der vorangehenden Ansprüche, wobei die Deckschicht aus einem Lösungsmittel, ausgewählt aus Glycerin und Monoalkylglycerinethern, und einem synthetischen Harz gebildet wird, das mit dem Lösungsmittel kompatibel ist.

5. Schicht nach Anspruch 4, wobei die Deckschicht weiterhin einen Absorber für ultraviolettes Licht und ein Wachs umfaßt.

6. Schicht nach Anspruch 4 oder Anspruch 5, wobei das synthetische Harz Acrylonitril-Butadien-Styrol-Harz ist.

## Revendications

1. Feuille pour distinguer si un traitement de stérilisation à plasma de peroxyde d'hydrogène a été effectué, ladite feuille comprenant un substrat, une couche à fonction d'indicateur déposée sur ledit substrat et une couche de revêtement déposée sur ladite couche à fonction d'indicateur, dans laquelle ladite couche à fonction d'indicateur comprend un colorant qui peut changer sa couleur quand il est mis en contact avec du peroxyde d'hydrogène ou un plasma dérivé du peroxyde d'hydrogène, et un composé amine organique qui ne s'évapore pas dans les conditions ambiantes, choisi parmi les mono amines aliphatiques supérieures, les mono amines aliphatiques supérieures hydroxy et la triéthanol amine, et dans laquelle ladite couche de revêtement est perméable à la vapeur de peroxyde d'hydrogène ou au plasma dérivé du peroxyde d'hydrogène, et est en outre transparente ou semi-transparente de telle manière qu'un changement de couleur du colorant est visible à travers ladite couche de revêtement.

2. Feuille selon la revendication 1, dans laquelle le colorant est un indicateur de pH qui a un intervalle de transition dans la plage de pH de 5,5 à 9,0, et dans laquelle le composé amine organique est la triéthanol amine.

3. Feuille selon la revendication 2, dans laquelle le colorant est le rouge phénol.

4. Feuille selon l'une quelconque des revendications précédentes, dans laquelle ladite couche de revêtement est formée d'un solvant choisi parmi le glycérol et les mono alkyl glycérol éthers, et une résine synthétique qui est compatible avec ledit solvant.

5. Feuille selon la revendication 4 dans laquelle ladite couche de revêtement comprend en outre un absorbeur de lumière ultraviolette et une cire.

6. Feuille selon la revendication 4 ou la revendication 5, dans laquelle la résine synthétique est la résine acrylonitrile-butadiène-styrène.
